# EUROPEAN PATENT APPLICATION

(11) **EP 0 731 176 A2**
(43) Date of publication of application: **11.09.1996**
(21) Application number: 96103567.2
(22) Date of filing: 07.03.1996
(51) Int. Cl.: C12Q 1/68

(54) **Method of gene duplication and amplification**

(30) Priority: 07.03.1995 JP 74616/95
(71) Applicant: NIPPON ZOKI PHARMACEUTICAL CO., LTD., Chuo-ku, Osaka (JP)
(72) Inventor: Imai, Hideki, c/o Inst. of Bio-Active Science, Yashiro-cho, Katoh-gun, Hyogo (JP)
(74) Representative: Kolb, Helga, Dr. Dipl.-Chem.

(57) **Abstract**

An object of the present invention is to offer a method for the duplication and also for the amplification of gene wherein the base sequence is ambiguous.

The constitution of the present invention is a method for duplication of gene, characterized in that, DNA which is complementary to RNA or double-stranded DNA of gene wherein the base sequence is ambiguous is dissociated into single-stranded DNA, then primer is partially bonded to a nonspecific position of the single-stranded DNA, DNA moiety having a complementarity to the single-stranded DNA is synthesized by DNA polymerase using said bonded primer as a starting point of the DNA synthesis and the above-mentioned operation is repeated twice or more whereby the nonspecific moiety of the gene is duplicated and also is a method of amplification of said gene using a PCR method thereafter.

The merit of the present invention is that gene wherein the base sequence is ambiguous can be duplicated and amplified and that it can be utilized for elucidation of unknown pathogenic microorganisms, for diagnosis of said infectious disease and for development of vaccine therefor.

## Description

The present invention relates to a method for the duplication of a gene wherein the base sequence is ambiguous by means of duplicating the nonspecific moiety of the gene; to a method for the amplification of the gene wherein the base sequence is ambiguous by means of amplifying the nonspecific moiety of the duplicated gene; to a method for the elucidation of the base sequence of the gene wherein the base sequence is ambiguous by elucidating the base sequence of each moiety of the amplified gene; to a method for the detection of unknown living organisms having the gene wherein the base sequence is ambiguous; to a duplicate of the nonspecific moiety of the gene wherein the base sequence is ambiguous obtained by the above-mentioned duplicating method; and to an amplified product of the nonspecific moiety of the gene wherein the base sequence is ambiguous obtained by the above-mentioned amplifying method.

Since a polymerase chain reaction (PCR) using a heat-resisting DNA synthetic enzyme was reported by Saiki, et al. in 1985, it has been possible to easily amplify in vitro the microorganism-derived DNA or RNA in small quantities existing in the sample derived from living organisms such as body fluid (e.g. blood, plasma, lymph, saliva and cerabrospinal fluid) and an extract from tissues.

Many patent applications have been filed already in Japan for a PCR method and the related area. Example of them relating to the representative fundamental technique are Laid-Open Patent Publications Sho-63/102677, Hei-02/000434, Hei-03/180178, Hei-03/180181 and Sho-62/240862. Among the inventions mentioned in those patent publications, those which are disclosed in the four publications from Sho-63/102677 to Hei-03/180181 constitute a series of inventions based upon the invention mentioned in Sho-63/102677 and relate to heat-resisting enzymes used for the PCR and the methods of using them. Incidentally, the invention mentioned in Sho-62/240862 relates to an apparatus used for the PCR and also to a method of using said apparatus. As hereunder, the inventions mentioned in Sho-63/102677 and Sho-62/240862 will be briefly explained.

Sho-63/102677 mentions the following inventions. They are a purified and thermally stable enzyme which catalyzes the binding of nucleotide triphosphate for forming a nucleic acid chain which is complementary to the template chain of nucleic acid (claim 1); gene which codes for DNA polymerase (claim 2); plasmid which is selected from pFC85 and pFC83 (claim 13); bacteriophage CH35; Taq #4-2 (claim 14); a stable enzyme composition containing a specific enzyme in a specific buffer (claim 15); a method comprising specific steps for the amplification of at least one kind of specific nucleic acid sequence contained in nucleic acid or a mixture of nucleic acids (claims 19 and 20); a method comprising specific steps for the identification of the presence or the absence of at least one specific nucleic acid sequence in the sample containing nucleic acid or a mixture of nucleic acids or for the discrimination of two different sequences in the above-mentioned samples (claims 21 and 22); a method comprising specific steps for the identification of the presence or the absence of at least one nucleic acid sequence change in one or more nucleic acid(s) contained in the sample (claims 23, 24, 25, 26 and 27); a method comprising specific steps for the cloning of one or more specific nucleic acid sequence(s) contained in nucleic acid or a mixture of nucleic acids to a cloning vector (claims 28, 29, 30 and 31); and an amplified nucleic acid sequence from nucleic acid or a mixture of nucleic acids having plural copies with a specific sequence produced by specific amplifying steps (claim 32).

Sho-62/240862 mentions the following inventions. They are an apparatus for conducting a temperature circulation of a reaction mixture in a PCR comprising specific steps (claim 1); an apparatus for conducting an automated amplification of at least one specific nucleic acid sequence comprising specific steps (claims 4 and 7); and a method for the amplification of at least one specific nucleic acid sequence comprising specific steps (claims 8 and 13).

Incidentally, when nucleic acid chain is contacted with a primer according to the invention (a PCR method) which is mentioned in Sho-63/102677, a hybridization is carried out at the temperature which is higher than a certain lower limit for hybridizing the primer at the specific moiety where the base sequence of nucleic acid chain is known as, for example, mentioned in the step (a) [from lines 5-3 from bottom of the upper right section of page 2] of claim 19 reading ".... the above-mentioned contact is carried out at the temperature whereby the hybridization of each primer to its complementary nucleic acid sequence is promoted;".

Thus, with regard to the above-mentioned temperature (annealing temperature), there are descriptions in the specification reading ".... conducted at about 45-58°C" (line 3, lower right section, page 14); ".... about 35-65°C or higher and, preferably, about 37-60°C" (lines 5-6, upper left section, page 29); and "in the case of Taq polymerase, .... it is lowered to about 37°C and, preferably, to about 45-58°C" (lines 5-3 from bottom, lower left section, page 29). The annealing temperature in the examples of said patent are as follows. Thus, they are 37°C in Example 1 [line 6, upper left section, page 44; gene human β-globulin; annealing time: three minutes]; 37°C [lines 5-8, upper left section, page 45; first sample, gene human β-globulin (Molt 4); annealing time: three minutes + two minutes)] and 37°C [lines 5-7, lower left section, page 45; second sample, gene human β-globulin (Molt 4); annealing time: one minute + one minute)] in Example 2; 37°C in Example 3 [lines 9-11, upper left section, page 47; gene human β-globulin (Molt 4, GM 2064; annealing time: thirty minutes + two minutes)]; 37°C in Example 7 [line 3 from bottom, upper left section, page 53; gene bacteriophage lambda DNA; annealing time: two minutes]; 35°C, 45°C and 55°C in Example 10 [from line 1 from bottom, upper left section to line 2, upper right section, page 54; gene human β-globulin (Molt 4, GM 2064), gene HIV-positive DNA (368H); annealing time (one minute + one minute)]; and 37°C [lines 5-4 from bottom, upper right section, page 55; first sample, cDNA of gene rabbit reticulocyte mRNA; annealing time: three minutes] and 37°C [from line 1 from bottom, lower right section, page 55 to line 2, upper left section, page 56; additional sample, cDNA of gene rabbit reticulocyte mRNA; annealing time: one minute + thirty seconds)] in Example 11.

Particularly in the above-mentioned Example 10, the annealing temperature is changed in three stages (35°C, 45°C and 55°C) and the influence caused by the change in the annealing temperature is checked. In lines 7-13, lower left section, page 54, there is a description reading "When the annealing temperature is 55°C, there was an improvement to an extent of one hundred times in terms of selectivity for Taq polymerase as compared with the case of 35°C and it was shown that, for the selectivity of Taq polymerase, a raised annealing temperature is important." This shows that, when the temperature is 55°C, a high specificity (selectivity) is achieved in a hybridization (annealing) of the primer to its complementary nucleic acid sequence.

In a PCR method, temperature for annealing is an extremely important condition. The following statement is found in a summary on a PCR method [Jikken Igaku (Experimental Medicine), Volume 8, No.9 (a special issue), page 36 (1990)]. Thus, "In order to detect the variation of human vitronectin gene for example, a condition of 55°C for two minutes was set from the sequence of a primer comprising 20 bases but, since several nonspecific bands were found, the condition was changed to 65°C for one minute whereupon the nonspecific bands completely disappeared."

Thus, it is a characteristic feature of a PCR method that the amplification of the moiety other than the desired gene and the contamination thereof are prevented whereby only aimed gene moiety is amplified. For such a purpose, primer should be specifically bonded at the specific moiety and an annealing under the condition of somewhat high temperature is essential.

When the temperature is higher, the primer is bonded only with the moiety having higher complementarity whereby the above-mentioned object can be achieved while, at a low temperature, it is nonspecifically bonded at the moiety having a low complementarity too whereby contamination of the gene moieties which are out of the object increases.

There are many literatures which usually give an annealing temperature around 55°C.

Accordingly, it is necessary for securing a high specificity (selectivity) in the PCR method that the annealing temperature is made higher than a certain temperature. From the descriptions in the above-mentioned patents relating to the fundamental invention, it is noted that such a temperature is not lower than 35°C.

The PCR method is capable of effectively amplifying the specific moiety of DNA but, for a specific hybridization of a primer to the specific moiety, it is necessary that the structure of the DNA which is to be amplified is previously elucidated. Cloning of gene is usually conducted for elucidating the base sequence of gene which requires the gene in certain amount. For such a purpose, it is necessary that the microorganism (such as virus) wherefrom the gene is derived is separated from the sample and is cultured. Therefore, when the amount of the microorganism in the sample is too small whereby it is unable to be separated or cultured, then elucidation of the base sequence of the gene is quite difficult. To be more specific, samples derived from unborn children, newborn babies, infants and patients suffering from special diseases are difficult to obtain in an sufficient amount except special cases.

The present invention is to solve the problems which have been difficult to conduct in the above-mentioned prior art and, unlike the conventional PCR method which is a method of amplifying the known base sequence, an object of the present invention is to offer a method for the duplication and the amplification of the base sequence applicable to the gene wherein the base sequence cannot be elucidated due to the small amount and also it is to offer various uses thereof. To be more specific, objects of the present invention are as follows.
a) Method for the duplication of the gene wherein the base sequence is ambiguous;
b) Method for the amplification of the gene wherein the base sequence is ambiguous;
c) Method for the elucidation of base sequence of the gene wherein the base sequence is ambiguous;
d) Method for the identification of unknown living organism having the gene wherein the base sequence is ambiguous;
e) Duplicate of the nonspecific moiety of the gene wherein the base sequence is ambiguous; and
f) Amplified product of the nonspecific moiety of the gene wherein the base sequence is ambiguous.

### [Means to solve the problems]

Thus, the method for the duplication of the gene in accordance with the present invention is characterized in that DNA which is complementary to RNA or double-stranded DNA of gene wherein the base sequence is ambiguous is dissociated into single-stranded DNA, then primer is partially bonded to a nonspecific position of the single-stranded DNA, DNA moiety having a complementarity to the single-stranded DNA is synthesized by DNA polymerase using said bonded primer as a starting point of the DNA synthesis and the above-mentioned operation is repeated twice or more whereby the nonspecific moiety of the gene is duplicated as a DNA having a base sequence corresponding to the above-mentioned primer (hereinafter, this method will be referred to as "i").

The method of the amplification of gene wherein the base sequence is ambiguous in accordance with the present invention is characterized in that the duplicated double-stranded DNA is dissociated into a single-stranded DNA after the method (i), then the primer which is same as that used in the above-mentioned method (i) is bonded at a specific position of the single-stranded DNA of the duplicated gene, DNA moiety having a complementarity to the single-stranded DNA is synthesized by DNA polymerase using said bonded primer as a starting point of the DNA synthesis and the above-mentioned operation is repeated twice or more whereby the nonspecific moiety of the gene is amplified (hereinafter, this will be referred to as "ii").

To be more specific, the above-mentioned method (i) for the duplication of the gene wherein the base sequence is ambiguous is characterized in that:
(a) two or more kinds of primers, reaction substrate, DNA polymerase and essential metal salt are added to a sample which contains gene wherein the base sequence is ambiguous in a form of DNA;
(b) the mixture is heated at a denaturing temperature so that the double-stranded DNA of the gene is dissociated into a single-stranded DNA;
(c) it is kept at an annealing temperature of lower than 35°C so that each of the primers is bonded to the nonspecific positions of the single-stranded DNA;
(d) it is heated at an elongating temperature so that the DNA moiety having a complementarity to each single-stranded DNA is synthesized by DNA polymerase using each of the primers partially bonded at the nonspecific positions of the single-stranded DNA as starting points of the DNA synthesis; and
(e) the steps (b), (c) and (d) are repeated twice or more whereby the nonspecific moiety of the gene is duplicated as DNA having the base sequence corresponding to the above-mentioned primers at the end (hereinafter, this method (a)-(e) will be referred to as "iii").

To be more specific, the above-mentioned method (ii) for the amplification of the gene wherein the base sequence is ambiguous is characterized in that, succeeding to the method of claim 3,
(f) it is heated at a denaturing temperature so that the duplicated double-stranded DNA is dissociated into a single-stranded DNA; then
(g) it is kept at an annealing temperature of 35°C or higher so that the primer which is the same as that used in the above-mentioned method (iii) is bonded at the specific position of the single-stranded DNA of the duplicated gene; then
(h) it is heated at an elongating temperature so that a DNA moiety having a complementarity to each single-stranded DNA is synthesized by DNA polymerase using each of the primers bonded at the specific positions of the duplicated single-stranded DNA as a starting point of the DNA synthesis; and
(i) the above-mentioned steps of (f), (g) and (h) are repeated for desired times whereby the nonspecific moieties of the gene are amplified (Hereinafter, this method (f)-(i) is referred to as "iv").

With regard to the primer used in the method (i) and in the step (a) of the method (iii) which are mentioned hereinabove, any kind of primer may be used because the base sequence of the gene which is to be duplicated is ambiguous. Thus, primers which have been used in the conventional PCR method may be suitably selected and used.

As mentioned later, the characteristic feature of the present invention is that an annealing is conducted at low temperature so that the primer is randomly bonded at the nonspecific moiety of the gene wherein the base sequence is ambiguous and then the duplication of the gene is conducted using said bonded place as a starting point. Accordingly, any kind of primer can be used. With regard to the length of the primer, that having some 20 bases (about 15-30 bases) can be preferably used due to the exclusion of the bond between primers and the linear bond with nucleic acid chain like in the case of the conventional PCR method.

With regard to the kind of the primer used, it is fundamentally preferred to use about 2-10 kinds when the operation of gene analysis which is conducted after the duplicated gene pieces is taken into consideration and, practically, about 5 kinds (4-7 kinds) are easily utilized.

With regard to the reaction substrate, DNA polymerase and essential metal salts which are used in the duplicating and amplifying methods of the present invention, those which have been used in the conventional PCR method may be suitably selected and used too. Thus, examples of the reaction substrate applicable are deoxyadenosine triphosphate (dATP), deoxycytidine triphosphate (dCTP), deoxyguanosine triphosphate (dGTP) and deoxythymidine triphosphate (dTTP) [or thymidine triphosphate (TTP)] while examples of the applicable metal salt for activating the DNA polymerase are magnesium salt and manganese salt. With regard to the reaction solution for conducting the method of the present invention, there is no need of using a special reaction solution but those which have been used in the conventional PCR method may be used. Thus, a suitable buffer containing, for example, potassium chloride, gelatin or other additives if necessary may be used.

The annealing temperature in the method (i) and in the step (c) of the method (iii) which are mentioned hereinabove is an important characteristic feature of the present invention. In order to bond a primer to a nonspecific position of a single-stranded DNA obtained by a dissociation of double-stranded DNA of the gene wherein the base sequence is ambiguous or DNA which is complementary to RNA, it is necessary to conduct the annealing at the temperature of lower than 35°C. When the annealing temperature is 35°C or higher, the primer is bonded only at the moiety having a very high homology to its base sequence and, therefore, a random bonding of the primer to the nonspecific position of the gene DNA which is an object of the present invention cannot be achieved. Accordingly, the annealing temperature in the present invention is lower than 35°C and, preferably, it is not higher than 30°C or, more preferably, not higher than 25°C for securing the sufficient nonselectivity to the single-stranded DNA and from a practical standpoint as well. With regard to the lower limit of the annealing temperature, the duplicating method of the present invention can be carried out provided that the temperature is within a range where the reaction solution is not frozen.

When the numbers of repeating the steps (b), (c) and (d) are two or more, it is possible to obtain a DNA duplicate having the base sequence corresponding to the primers used at both terminals. In that case, the more the repeating numbers, the more the kinds of the duplicate. However, when the kinds of the duplicate are too many, the identifying operation (such as that by a Southern blotting) of the gene thereafter becomes complicated. Therefore, although the repeating numbers may be suitably set, it is usually twice whereby the object of the present invention can be achieved.

In accordance with the duplicating method of the present invention as mentioned above, it is possible to obtain a DNA duplicate having the base sequence at the terminals corresponding to the primers used at both terminals. The DNA moiety which is sandwiched between the primer moieties at both terminals is the nonspecific moiety of the unknown gene. Then, in order to elucidate the base sequence of this unknown gene moiety, it is necessary to amplify the DNA duplicate obtained by the above duplicating method to the amount whereby the analysis is possible. The base sequence at the both terminals of said DNA duplicate is known (it is a base sequence corresponding to the used primer) and, therefore, conventional PCR method is applied to this whereby said DNA fragments can be amplified.

Actually, it can be conducted by repeating the above-mentioned steps (f), (g) and (h) for desired times.

Naturally, the primers used for this amplifying method are one or two primer(s) supposed to be present at both terminals of the resulting DNA duplicate. Various reaction conditions such as denaturing temperature, annealing temperature, elongating temperature, reaction substrates, DNA polymerase, essential metal salts, buffers, additives and repeating numbers of the amplifying steps may be suitably selected from those used for the common PCR method and carried out by the same manner as in the conventional method.

The gene which is amplified by the amplifying method of the present invention is separated by, for example, electrophoresis and then the base sequence of each moiety is elucidated by common methods whereby the base sequence of the starting gene can be elucidated. Further, when a part of or all of the base sequence of each moiety of the gene which is amplified by the above-mentioned method is tested or elucidated, it is possible to detect the unknown living organism (e.g. unknown microorganism such as virus) wherefrom the starting gene is derived. Furthermore, it is possible by the duplicating method of the present invention to obtain a duplicate of the nonspecific moiety of the gene wherein the base sequence is ambiguous and it is also possible by the amplifying method of the present invention to obtain an amplified product of the nonspecific moiety of the gene wherein the base sequence is ambiguous.

### I. Basic Principles.

1) In the base sequence of gene, there is one moiety every 1,400 to 1,500 bp in average which has about 50% homology to the base sequence of the primer (in case it comprises about 20 bases) and where two or more continuous bases at the 3'-side (which is a starting point for the DNA synthesis) of the primer have a 100% homology. When annealing temperature is lowered in bonding a primer to a single-stranded DNA obtained by a dissociation of the double-stranded DNA of gene or the DNA which is complementary to RNA, the primer is partly bonded to said moiety (the nonspecific position of the single-stranded DNA) even if the base sequence of the gene is ambiguous. Thus, when the DNA is synthesized and elongated using the randomly bonded primer as a starting point, the nonspecific moiety of the gene can be duplicated.
2) When the above-mentioned gene duplication is conducted using plural kinds of primers and then the gene amplification is conducted using each two kinds of primers, it is possible to extract some specific groups from the duplicated DNA groups having the terminal base sequence (corresponding to the base sequence of each primer) in a complicated combination.

### II. General Operations.

### 1) Extraction of Nucleic Acid.

(1) Selection of the Sample.
   For example, a plasma sample which is supposed to contain unknown virus particles (X) is used.
(2) Reverse Transcription Reaction (conducted if necessary).
   cDNA is prepared from RNA by a common reverse transcription reaction using a reverse transcriptase. When it is clear that the sample contains DNA only, this reaction may be omitted.

With regard to the primer, five kinds such as a, b, c, d and e are used.

### 2) Gene Duplication.

### (1) Duplicating Operation.

Primers (a, b, c, d and e) are used and they are bonded to the nonspecific positions of a single-stranded DNA obtained by a thermal dissociation of double-stranded DNA (annealing temperature: not higher than 25°C for example) and then each operation for DNA synthesis is conducted. Some cycles (two cycles for example) are carried out.

### (2) Duplicate M (Mixture).

Since five kinds of primers (a, b, c, d and e) are used, duplicates can be classified into 15 kinds, i.e. M1, M2, M3, ... and M15 [MX (X = 1-15)]. The MX is defined as shown in the following Table 1 according to the combination of the primer terminals in the duplicate.

**Table 1**

| | a | b | c | d | e |
|---|---|---|---|---|---|
| a | 1 | 2 | 3 | 4 | 5 |
| b | - | 6 | 7 | 8 | 9 |
| c | - | - | 10 | 11 | 12 |
| d | - | - | - | 13 | 14 |
| e | - | - | - | - | 15 |

It is noted from Table 1 that, for example, M1 has terminal primers of a---a, M2 has terminal primers of a---b. Incidentally, each MX is a mixture of plural moieties of unknown gene having different length and base sequence sandwiched by one or two primer terminal(s).

### 3) Gene Amplification.

### (1) Amplifying Operation.

One preferred embodiment is that, first, the gene amplification from the gene product is carried out using the five kinds of primers which are same as those used for the gene duplication [corresponding to the above-mentioned steps (f)-(i)]. This amplifying method can be conducted by the same manner as in the conventional PCR method and, for example, the annealing temperature is set at 35°C or higher. The reason why the same five kinds of primers (a, b, c, d and e) as in the case of gene duplication are used for the amplifying operation is that the amount of the duplicate is increased to such an extent that is sufficient for each amplifying operation of the duplicates using two or one kind(s) of primer(s) which will be conducted in the next step. This amplifying operation is conducted in several tens cycles as same as in the conventional PCR method. For example, the above-mentioned steps (f)-(h) are repeated for 30 cycles but, for achieving a sufficient amplification, said 30 cycles may be repeated once again.

Then, two or one of the five kinds of primers (a, b, c, d and e) are/is used to conduct a gene amplification from the duplicated product M once again whereby each of M1, M2, M3, ... and M15 is amplified. For example, when primer a is used, M1 can be amplified and, when primers a and b are used, M2 can be amplified individually. Such an individual amplifying operation can be conducted repeatedly in several tens cycles (for example, 30 cycles) as same as in the case of the conventional PCR method.

### 4) Detection of Virus (X) Gene.

Common method such as a Southern blotting is used.

### 〈Southern Blotting Method〉

M1, M2, M3, ... and M15 are blotted.

Amplified products (m1, m2, m3, ... and m15) derived from the control are used as probes.

"Control" is a sample containing no unknown virus and the like which are not intended to be detected such as plasma in a healthy and normal state.

It is strongly suggested that the DNA fragment which is not identified by the probe is a part of the gene derived from the unknown virus (X).

### 5) Elucidation of Base Sequence of Gene Derived from X.

Gene which is suggested to be derived from X is taken out in a pure form by a common operation (such as extraction), then it is subjected to an amplification by a PCR method using a suitable primer among the above-mentioned five kinds of primers (a, b, c, d and e) and the base sequence is elucidated by a common method.

### Brief Explanation of the Drawings:

Fig. 1 is a flow chart of the steps for conducting the method for the amplification of gene for investigating the influence of the annealing temperature.

Fig. 2 shows a relationship between the annealing temperature and the amount of the amplified product in an experiment (Example 1) when the amplified product derived from a template DNA (pBR322) with 53 pg is detected by a Southern method followed by quantifying it by a densitometer.

### [Examples]

The present invention will be further illustrated by way of the following examples. Those examples are given for explaining the present invention and are not intended to limit the present invention thereto.

Example 1. Investigation of Annealing Temperature.

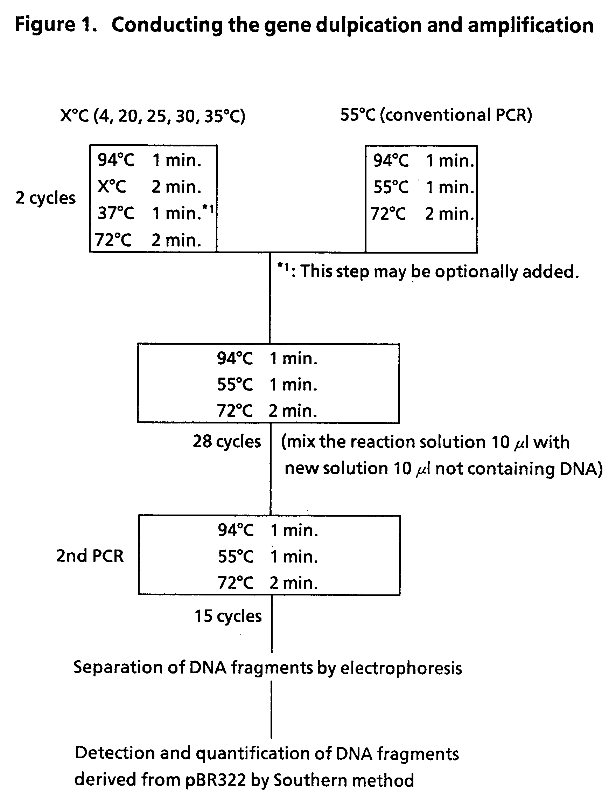

Influence of the annealing temperature on amplification of small amount of DNA was investigated. Flow chart of the steps which are conducted for the gene amplification is given in Fig. 1.

### 〈Method〉

### (1) Composition of Reaction Solution.

A reaction solution having the following composition was used.
50 mM of Tris-HCl (pH: 8.3);
6 mM of MgCl₂;
40 mM of KCl;
2.5 mM of dATP;
2.5 mM of dTTP;
2.5 mM of dGTP;
2.5 mM of dCTP;
10% (w/v) of dimethyl sulfoxide (DMSO);
1.5 µM of primer (20 mer; including the five kinds which will be mentioned later);
0.5 unit of heat-resisting DNA polymerase (Taq polymerase); and
0-0.53 ng of 0-10⁹ copy; pBR322 (template DNA).

### (2) Conducting the Gene Amplification.

### I. Duplicating Operation.

a) An Example of Investigations on Annealing at Low Temperature.
   Annealing temperature (T°C) was varied at T = 4, 20, 25, 30 and 35(°C) and, for each of them, the reaction under the following condition was conducted in two cycles.
   at 94°C for one minute;
   at T°C for two minutes;
   [at 37°C for one minute; when T (°C) was 20 (°C), the influence of this step was checked. Thus, for fearing that the primer may be detached if the temperature was raised at a time from 20°C to 72°C, the propriety of a stepwise rise in the temperature by keeping the sample at 37°C for a certain period was investigated.]; and
   at 72°C for two minutes.
b) Annealing at 55°C by Common PCR Method (Control Example).
   at 94°C for one minute;
   at 55°C for one minute; and
   at 72°C for two minutes.

### II. First Amplifying Operation.

Succeeding to the above-mentioned a) and b), the first amplifying operation (the first PCR) was conducted for each of the cases under the following condition for 28 cycles.
at 94°C for one minute;
at 55°C for one minute; and
at 72°C for two minutes.

### III. Second Amplifying Operation.

In each case, 10 µl of the reaction solution after the completion of the first amplifying operation was mixed with 10 µl of a new reaction solution containing no DNA and then subjected to the second amplifying operation (the second PCR) under the following condition for 15 cycles.
at 94°C for one minute;
at 55°C for one minute; and
at 72°C for two minutes.

### IV. Separation of DNA Fragments.

In each case, the reaction after completion of the second amplifying operation was subjected to a common electrophoresis whereupon the DNA fragments were separated.

### V. Identification and Quantitative Determination.

In each case, DNA fragments derived from pBR322 were identified and determined from the separated DNA fragments using a Southern method.

### 〈Results and Discussions〉

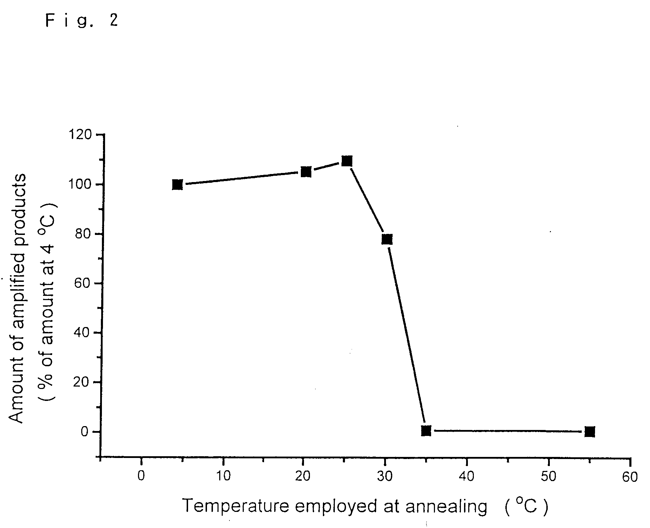

Fig. 2 is a graph in which the amplified product derived from a template DNA (pBR322) of 53 pg was identified by a Southern method and the result is quantified by means of a densitometer. Abscissa shows the temperature used for the annealing (annealing temperature given in terms of °C) while ordinate shows the amount of the amplified product (in terms of an amplified rate in % when the amount at 4°C was defined as 100%). When the annealing temperature was 35°C or higher, the amplified product was not more than 1/100 of the amount at 4°C while, when it was lower than 35°C, especially 30°C or lower, there was a significant increase in the amplified product and, at 25°C or lower, the amount was nearly constant. From the above results, it is noted that the temperature suitable for the amplification of small amount of unknown gene is lower than 35°C, preferably not higher than 30°C and, more preferably, not higher than 25°C.

With regard to the step of keeping at 37°C for one minute after annealing at low temperature, it was found that said step did not affect the amount of the amplified product because the amplifying rate when this step was conducted was 100% while that when this step was not conducted was 97%.

### Example 2.

In this Example 2, human immunodefficient virus-1 (HIV-1) was used as an unknown virus (X) and it was checked whether this HIV-1-derived gene was able to be identified by the method of the present invention.

### 〈Method〉

The cultured liquid containing human immunodefficient virus-1 (HIV-1) was diluted, added to human plasma and the resulting sample for the measurement was filtered through a filter of 0.2 µm pore size. The filtrate was diluted with 50 mM Tris buffer (pH: 7.5), an equivalent amount of water-saturated phenol was added thereto followed by mixing vigorously and the aqueous layer was centrifuged. This was mixed with chloroform, centrifuged and the aqueous layer was collected and freeze-dried. Sterilized distilled water was added to the freeze-dried sample so that the sample was completely dissolved. To a part of the solution were added five kinds of single-stranded DNA comprising 20 free bases entirely unrelated to the base sequence of HIV-1 (as primers); deoxyadenosine triphosphate (dATP), deoxycytidine triphosphate (dCTP), deoxyguanosine triphosphate (dGTP) and thymidine triphosphate (TTP) (as reaction substrates); Tris buffer (pH: 8.3); RNAase inhibitor; magnesium chloride and potassium chloride; dithiothreitol; and reverse transcriptase. The resulting mixture was allowed to stand at room temperature for 20 minutes followed by keeping at 37°C for 60 minutes to conduct a reverse transcription reaction. The primers used in this example were as follows.

### [Base Sequences of the Primers; 5'-terminal---3'-terminal]

(1) CAATTGGGGTTGAGCTCACA
(2) ACAGAGTCCTTTTCTGACAT
(3) ACCAACTATTGCTTCAGCTC
(4) CCATTCATTTGTCTTTTTAG
(5) CACATCAGTTTTATTCTTGG

Then duplicating and amplifying reactions of the gene were conducted. They comprised the duplicating/amplifying reactions in three steps and sixty cycles. First, a part of the reverse transcription reaction solution was mixed with Tris buffer (pH: 8.3), magnesium chloride, potassium chloride, dithiothreitol, dimethyl sulfoxide and heat-resisting DNA polymerase (DNA synthetic enzyme) and subjected to two cycles of the reaction (gene duplicating reaction). One cycle of the reaction comprised heating at 94°C for one minute, at 20°C for two minutes and at 72°C for two minute.

After that, a reaction comprising heating at 94°C for one minute, at 55°C for one minute and at 72°C for two minutes was conducted in 28 cycles. A part of it was then mixed with deoxyadenosine triphosphate (dATP), deoxycytidine triphosphate (dCTP), deoxyguanosine triphosphate (dGTP), thymidine triphosphate (TTP), Tris buffer (pH: 8.3), magnesium chloride, potassium chloride, dithiothreitol, dimethyl sulfoxide and heat-resisting DNA synthetic enzyme (five kinds of primers may be added thereto) and the mixture was subjected to 30 cycles of the reaction (gene amplifying reaction). Each reaction cycle comprised heating at 94°C for one minute, at 55°C for one minute and at 72°C for two minutes.

A positive control test was conducted as follows. Thus, to the same amount as above of the solution prepared by dissolving the same freeze-dried material as above in distilled water were added two kinds of single-stranded DNA having a 100% complementarity to the base sequence of HIV-1 (as primers) (a primer-pair in which one has a sequence of sense of HIV-1 gene while another has a sequence of antisense of HIV-1 gene); deoxyadenosine triphosphate (dATP), deoxycytidine (dCTP), deoxyguanosine triphosphate (dGTP) and thymidine triphosphate (TTP) (as reaction substrates); Tris buffer (pH: 8.3); RNAase inhibitor; magnesium chloride and potassium chloride; dithiothreitol; dimethyl sulfoxide; and reverse transcriptase and then the mixture was subjected to a reverse transcription reaction at 37°C for 60 minutes.

The gene amplifying reaction comprised two steps in 60 cycles.

First, a part of the reverse transcription reaction solution was mixed with Tris buffer (pH: 8.3), magnesium chloride, potassium chloride, dithiothreitol, dimethyl sulfoxide and heat-resisting DNA polymerase and the mixture was subjected to 30 cycles of the reaction (one cycle comprising heating at 94°C for one minute, at 55°C for one minute and at 72°C for two minutes). A part of the resulting reaction solution was further mixed with deoxyadenosine triphosphate (dATP), deoxycytidine triphosphate (dCTP), deoxyguanosine (dGTP) and thymidine triphosphate (TTP); Tris buffer (pH: 8.3); magnesium chloride and potassium chloride; dithiothreitol; dimethyl sulfoxide; and heat-resisting DNA polymerase and subjected to 30 cycles of the reaction. One cycle comprised heating at 94°C for one minute, at 55°C for one minute and at 72°C for two minutes.

As a negative control test, the same experiment as in the positive control test was conducted starting from human plasma to which no human immunodefficient virus-1 (HIV-1) was added.

The material in which the gene amplifying reaction was completed was separated by means of a 6% polyacrylamide electrophoresis, further transcribed to a Nylon membrane, hybridized with DNA having the same base sequence as HIV-1 and being labeled with ³²P and subjected to an autoradiography whereupon the amplified HIV-1 gene was detected.

Each one or two out of the five kinds of the primers used for the gene amplification was/were combined and, again, gene amplification was conducted using a material containing the HIV gene wherein the gene amplification reaction was completed and also a material for a negative control (resulting in the gene products each of which contained fifteen kinds of primer-pair). All of them were separated by means of a 6% polyacrylamide gel electrophoresis and DNA fragments in the gel were dyed.

The DNA fragments in the gel were transcribed to polyamide (Nylon) membrane or the like and detection of the DNA fragments was conducted by a Southern method using a probe in which the amplified product derived from the negative control was labeled with ³²P. The DNA fragments which were not detected by the Southern method among the amplified products derived from the HIV gene developed on a 6% polyacrylamide gel were believed to be specific to said amplified products and, therefore, the DNA fragments were extracted, purified, subjected to a gene amplification (PCR method) again using the primers which were used for the previous amplification and the base sequence was elucidated by a common method.

### 〈Results〉

When the number of the infectious HIV-1 particle is one (corresponding to 100-1,000 noninfectious particles), the gene amplification from the particle of one-tenth of that (corresponding to 10-100 noninfectious particles) was observed in accordance with the method of the present invention. On the other hand, in the case of a positive control experiment (conventional PCR method), the gene amplification from one-hundredth of that (corresponding to 1-10 noninfectious particles) was observed. The amplifying rate according to the present invention as judged from the signals of autoradiography was not less than 100,000-fold and a practically sufficient amplifying rate can be achieved. When the base sequence of the separated DNA fragment was checked, a high homology (not less than 94%) was found at the LTR (long terminal repeat) region of HIV-1 (HIV-IIIB strain). When the region where primer was thought to be bonded was checked, a 55% homology with the primer used for the amplification was found and, in addition, the 3'-terminal of the primer was entirely identical with the sequence of HIV-1. In the conventional PCR method, a small amount of gene wherein the base sequence is ambiguous cannot be amplified and, therefore, elucidation of its base sequence after its separation is not possible. Consequently, it is understood that the present invention is extremely useful.

In the conventional PCR method, gene (DNA) having a known base sequence is a subject. Thus, the principle of the PCR method is that two kinds of primers which sandwich the aimed gene region are used whereby said region can be doubled by a single reaction and that said DNA polymerase reaction is repeated. As mentioned already, it is the characteristic feature of the PCR method that contamination with the moiety other than the aimed gene is prevented whereby only the aimed gene moiety is amplified. For such a purpose, primer should be specifically bonded at a specific moiety and an annealing at somewhat high temperature is essential. When the temperature is higher, the primer is bonded only at the moiety having higher complementarity whereby the above-mentioned object can be achieved while, at the lower temperature, the primer is nonspecifically bonded even at the moiety having lower complementarity whereby contamination of the gene moiety other than the aimed one increases.

The method of the present invention is for identifying the gene wherein the base sequence is ambiguous utilizing the amplification of gene outside the object which is to be avoided, if at all possible, in the conventional PCR method. Thus, an object of the present invention is that the annealing is conducted at low temperature so that primer is randomly bonded at nonspecific moiety of gene whereby nonspecific moiety of the unknown gene is duplicated and amplified.

Consequently, in accordance with the method of the present invention, gene can be duplicated and amplified using, for example, plasma or the like derived from a patient infected by unknown (unidentified) pathogenic microorganism as a material.

When the base sequence of the resulting DNA can be epidemiologically confirmed that it is derived from a microorganism which is specific to said infectious disease, it is now possible to diagnose the infection and also to prepare a vaccine for said infectious disease.

In addition, when the base sequence of each moiety of gene which is amplified by the method of the present invention is elucidated, it is possible to elucidate the base sequence of the gene wherein the base sequence is ambiguous. Moreover, when a part of or all of the base sequence of each moiety of the gene which is amplified by the method of the present invention is tested or elucidated, it is possible to identify the unknown living organism having gene wherein the base sequence is ambiguous. Accordingly, the present invention is useful as a method for elucidating the base sequence of gene wherein the base sequence is ambiguous and also for identifying the unknown living organism having gene wherein the base sequence is ambiguous.

Further, duplicated and amplified product of the nonspecific moiety of gene wherein the base sequence is ambiguous can be easily obtained by the method of the present invention and the product is useful as the subject for testing or analyzing the structure and the function of gene.

## Claims

1. A method for the duplication of genes, characterized in that DNA which is complementary to RNA or double-stranded DNA of a gene whose base sequence is ambiguous is dissociated into single-stranded DNA, then primer is partially bound to a nonspecific position of the single-stranded DNA, a DNA moiety having a complementarity to the single-stranded DNA is synthesized by DNA polymerase using said bound primer as a starting point of the DNA synthesis and the above-mentioned operation is repeated twice or more whereby the nonspecific moiety of the gene is duplicated as a DNA having a base sequence corresponding to the above-mentioned primer.

2. A method for the amplification of genes in which the base sequence is ambiguous, characterized in that the duplicated double-stranded DNA is dissociated into a single-stranded DNA according to the method of claim 1, then the primer which is the same as that used in claim 1 is bound at a specific position of the single-stranded DNA of the duplicated gene, a DNA moiety having a complementarity to the single-stranded DNA is synthesized by DNA polymerase using said bound primer as a starting point of the DNA synthesis and the above-mentioned operation is repeated twice or more whereby the nonspecific moiety of the gene is amplified.

3. A method for the duplication of gene, characterized in that
(a) two or more kinds of primers, reaction substrate, DNA polymerase and essential metal salt are added to a sample which contains gene wherein the base sequence is ambiguous in a form of DNA;
(b) the mixture is heated at a denaturing temperature so that the double-stranded DNA of the gene is dissociated into a single-stranded DNA;
(c) said mixture is kept at an annealing temperature of lower than 35°C so that each of the primers is bound to the nonspecific positions of the single-stranded DNA;
(d) said mixture is heated at an elongating temperature so that the DNA moiety having a complementarity to each single-stranded DNA is synthesized by DNA polymerase using each of the primers partially bound at the nonspecific positions of the single-stranded DNA as starting points of the DNA synthesis; and
(e) the steps (b), (c) and (d) are repeated twice or more whereby the nonspecific moiety of the gene is duplicated as DNA having the base sequence corresponding to the above-mentioned primers at the end.

4. A method for the amplification of genes in which the base sequence is ambiguous, characterized in that subsequent to steps (a) to(e) of claim 3,
(f) said mixture is heated at a denaturing temperature so that the duplicated double-stranded DNA is dissociated into a single-stranded DNA; then
(g) said mixture is kept at an annealing temperature of 35°C or higher so that the primer which is the same as that used in claim 3 is bound at the specific position of the single-stranded DNA of the duplicated gene; then
(h) said mixutre is heated at an elongating temperature so that a DNA moiety having a complementarity to each single-stranded DNA is synthesized by DNA polymerase using each of the primers bound at the specific positions of the duplicated single-stranded DNA as a starting point of the DNA synthesis; and
(i) the above-mentioned steps of (f), (g) and (h) are repeated for desired times whereby the nonspecific moieties of the gene are amplified.

5. A method according to claim 3 or claim 4, characterized in that, the steps (b), (c) and (d) are repeated twice.

6. A method for the elucidation of the base sequence of a gene in which the base sequence is ambiguous, characterized in that the base sequence of each moiety of the gene is amplified by the method of claim 4.

7. A method for the detection of unknown living organisms, characterized in that a part of or all of the base sequence of each moiety of the gene amplified by the method of claim 4 is tested or elucidated.

8. Duplicate of the nonspecific moiety of a gene in which the base sequence is ambiguous obtained by a method of claim 3.

9. Amplified product of the nonspecific moiety of a gene in which the base sequence is ambiguous obtained by a method of claim 4.
